# EUROPEAN PATENT SPECIFICATION

(11) **EP 2 552 540 B1**
(45) Date of publication and mention of the grant of the patent: **11.05.2016**
(21) Application number: 11713605.1
(22) Date of filing: 31.03.2011
(51) Int. Cl.: A61N 1/375, H01R 43/02

(54) **METHOD OF FABRICATING IMPLANTABLE PULSE GENERATOR USING WIRE CONNECTIONS TO FEEDTHROUGH STRUCTURES AND IMPLANTABLE PULSE GENERATORS**
VERFAHREN ZUR HERSTELLUNG EINES IMPLANTIERBAREN PULSGEBERS MIT DRAHTVERBINDUNGEN ZU DURCHFÜHRUNGEN UND IMPLANTIERBARER PULSGEBER
PROCÉDÉ DE RÉALISATION D'UN GÉNÉRATEUR IMPLANTABLE D'IMPULSIONS UTILISANT DES BRANCHEMENTS FILAIRES VERS DES STRUCTURES DE TRAVERSÉE, ET GÉNÉRATEURS IMPLANTABLES D'IMPULSIONS

(30) Priority: 31.03.2010 US 319682 P; 31.03.2010 US 319677 P; 30.12.2010 US 428519 P; 31.03.2010 US 319679 P
(43) Date of publication of application: 06.02.2013
(73) Proprietor: Advanced Neuromodulation Systems, Inc., Plano, TX 75024 (US)
(72) Inventor: MCGIBONEY, Ken, Frisco, TX 75034 (US); SMITH, Galen, L., Allen, TX 75002 (US); GAINES, Michael, Little Elm, TX 75068 (US); BOOGAARD, Jerome, Forest Grove, OR 97116 (US); GUTIEREZ, Eric, Bedford, TX 76021 (US)
(74) Representative: Schweiger, Martin
(86) International application number: PCT/US2011/030704
(87) International publication number: WO 2011/123627

(56) References cited:
- US-A- 4 220 814
- US-A- 5 272 283
- US-A- 5 571 146
- US-A1- 2006 221 543
- US-B1- 7 736 191

## Description

### TECHNICAL FIELD

This application is generally related to a method of fabricating an implantable pulse generator using wire connections to feedthrough structures.

### BACKGROUND

Neurostimulation systems are devices that generate electrical pulses and deliver the pulses to nerve tissue to treat a variety of disorders. Spinal cord stimulation (SCS) is the most common type of neurostimulation. In SCS, electrical pulses are delivered to nerve tissue in the spine typically for the purpose of chronic pain control. While a precise understanding of the interaction between the applied electrical energy and the nervous tissue is not fully appreciated, it is known that application of an electrical field to spinal nervous tissue can effectively mask certain types of pain transmitted from regions of the body associated with the stimulated nerve tissue. Specifically, applying electrical energy to the spinal cord associated with regions of the body afflicted with chronic pain can induce "paresthesia" (a subjective sensation of numbness or tingling) in the afflicted bodily regions. Thereby, paresthesia can effectively mask the transmission of non-acute pain sensations to the brain.

SCS systems generally include a pulse generator and one or more leads. A stimulation lead includes a lead body of insulative material that encloses wire conductors. The distal end of the stimulation lead includes multiple electrodes that are electrically coupled to the wire conductors. The proximal end of the lead body includes multiple terminals, which are also electrically coupled to the wire conductors, that are adapted to receive electrical pulses. The distal end of a respective stimulation lead is implanted within the epidural space to deliver the electrical pulses to the appropriate nerve tissue within the spinal cord that corresponds to the dermatome(s) in which the patient experiences chronic pain. The stimulation leads are then tunneled to another location within the patient's body to be electrically connected with a pulse generator or, alternatively, to an "extension."

The pulse generator is typically implanted within a subcutaneous pocket created during the implantation procedure. In SCS, the subcutaneous pocket is typically disposed in a lower back region, although subclavicular implantations and lower abdominal implantations are commonly employed for other types of neuromodulation therapies.
The pulse generator is typically implemented using a metallic housing that encloses circuitry for generating the electrical pulses, control circuitry, communication circuitry, a rechargeable battery, etc. The pulse generating circuitry is coupled to one or more stimulation leads through electrical connections provided in a "header" of the pulse generator. Specifically, feedthrough wires typically exit the metallic housing and enter into a header structure of a moldable material. Within the header structure, the feedthrough wires are electrically coupled to annular electrical connectors. The header structure holds the annular connectors in a fixed arrangement that corresponds to the arrangement of terminals on a stimulation lead.

US 5 272 283 discloses a cochlear prosthetic package having a cup-shaped titanium case with a ceramic plate. A plurality of hermetically-sealed feedthroughs are formed in the plate by sintering the plate with hollow metallic tubes located in plate holes and then sealing at least one end of each tube. An electrode cable connector is provided which allows removal and replacement of a case even in the presence of body fluids. A telemetry coil is contained in a metallic tube which extends out of the case; the coil-containing tube has its two ends hermetically-sealed to the case by ceramic insulating bushings.

US 2006/221543 A1 discloses a feedthrough terminal assembly for an active implantable medical device that utilizes an insert to establish a reliable electrical connection between capacitor electrode plates, via inner surface metallization of a capacitor aperture, and an associated terminal pin 10, which passes at least partially therethrough. The inserts are preferably resiliently flexible, such as a spring, to establish this connection. The insert also serves to establish a mechanical connection between the capacitor and the terminal pin.

US 5 571 146 discloses, for implantable medical devices, a system for welding electrically conductive ribbon, typically stainless steel, to electrically conductive wire, typically platinum, the melting point of steel being substantially lower than that of platinum.

US 4 220 814 discloses a terminal for introducing signals from an electrical terminal pin in a heart pacemaker to a terminal lead introduced into a patient's body.

### SUMMARY

In one embodiment, a method of fabricating an implantable pulse generator for generating electrical pulses for application to tissue of a patient. The method comprises providing one or more housing components adapted to house at least pulse generating and switching circuitry of the implantable pulse generator; providing a feedthrough assembly adapted to be coupled with the one or more housing components, the feedthrough assembly comprising an array of feedthrough pins for providing respective electrical connections from a hermetically sealed enclosure formed within the one or more housing components to a location exterior to the enclosure; providing a plurality of welding components with each welding component comprising a first portion adapted to engage a respective feedthrough pin and a second portion for engaging a conductor wire; performing one or more weld operations for each welding component to connect each welding component with a respective feedthrough pin and a respective conductor wire by applying a majority of welding energy directly to the welding components; wherein, within the enclosure, each feedthrough pin is electrically coupled to the pulse generating circuitry through the switching circuitry of the implantable pulse generator, wherein (i) each of the plurality of welding components is ferrule tube connector, (ii) each of the feedthrough pins are welded to a respective ferrule tube connector that circumscribes the corresponding feedthrough pin and (iii) each of the plurality of conductor wires is disposed within a slot of a corresponding ferrule tube connector with the respective conductor wire being welded to its corresponding ferrule tube connector.

In other embodiments, an implantable pulse generator device for stimulation of tissue of a patient is fabricated using one or more of the methods and/or one or more of the welding components disclosed herein.

An implantable pulse generator, comprises a housing for hermetically enclosing pulse generating circuitry and switching circuitry of the implantable pulse generator; a feedthrough assembly welded to the housing, the feedthrough assembly comprising a plurality of feedthrough pins extended from within an interior space within the housing to a location exterior to the housing, the feedthrough pins being electrically coupled to the pulse generating circuitry through the switching circuitry; an extension component comprising (i) a lead body extending away from the housing, wherein the lead body encloses a plurality of conductor wires enclosed within insulative material, and (ii) an electrical connector portion adapted to receive terminals of a stimulation lead, wherein the extension component is non-removably attached to the housing; and a plurality of welding components that each comprise a first portion welded to a corresponding feedthrough pin of the feedthrough assembly and at least one tab structure at least partially surrounding and welded to a corresponding conductor wire of the lead body.

### BRIEF DESCRIPTION OF THE DRAWINGS

FIG. 1 depicts a stimulation system according to one representative embodiment.
FIGS. 2A-2C respectively depict stimulation portions for inclusion at the distal end of a lead according to some representative embodiments.
FIGS. 3A-3E depict respective components for creating an electrical connection from within the housing of a pulse generator to wire conductors of a lead body according to one representative embodiment.
FIG. 4 depicts integration of an intermediate assembly including feedthrough structure with one or more housing component(s) of an implantable pulse generator according to one representative embodiment. FIG. 5 depicts another plurality of components for an intermediate assembly including feedthrough structure for integration with one or more housing component(s) of an implantable pulse generator according to one representative embodiment.
FIGS. 6 and 7 depict processing of a feedthrough pin according to one representative embodiment.
FIGS. 8-12 depict a series of structures (during various processing steps) during connection of a wire of a lead body to a feedthrough pin according to one representative embodiment.
FIGS. 13-15 depict electrical connection of a respective wire of a lead body to a feedthrough pin according to another representative embodiment.
FIGS. 16A-16F depict processing of a feedthrough pin during creation of an electrical connection with a conductor wire according to one representative embodiment.

### DETAILED DESCRIPTION

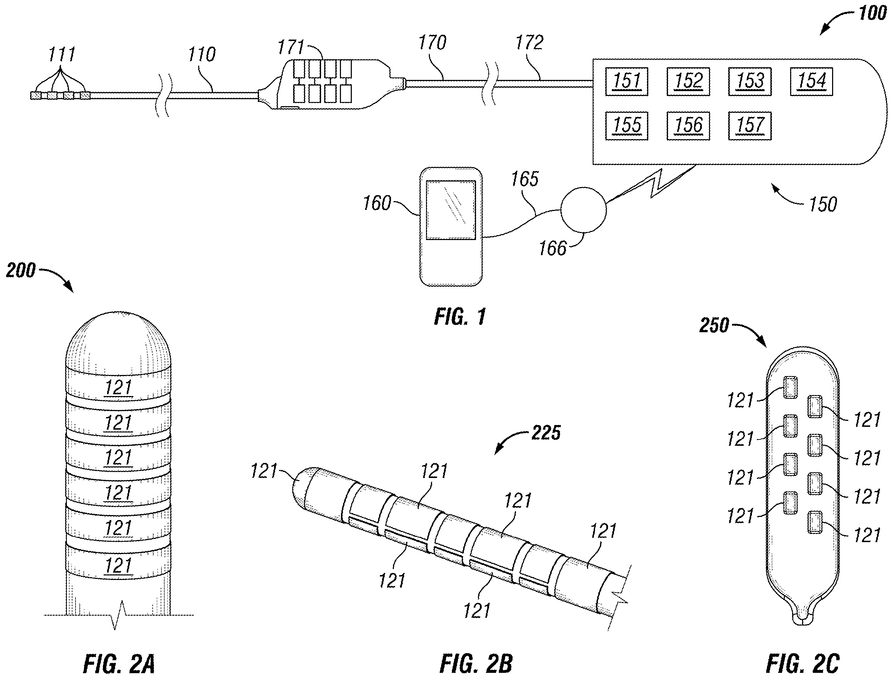

FIG. 1 depicts stimulation system 100 that generates electrical pulses for application to tissue of a patient according to one embodiment. For example, system 100 may be adapted to stimulate spinal cord tissue, peripheral nerve tissue, deep brain tissue, cortical tissue, cardiac tissue, digestive tissue, pelvic floor tissue, or any other suitable tissue within a patient's body.

System 100 includes implantable pulse generator 150 that is adapted to generate electrical pulses for application to tissue of a patient. Implantable pulse generator 150 typically comprises a metallic housing that encloses controller 151, pulse generating circuitry 152, charging coil 153, battery 154, far-field and/or near field communication circuitry 155, battery charging circuitry 156, switching circuitry 157, etc. of the device. Controller 151 typically includes a microcontroller or other suitable processor for controlling the various other components of the device. Software code is typically stored in memory of the pulse generator 150 for execution by the microcontroller or processor to control the various components of the device.

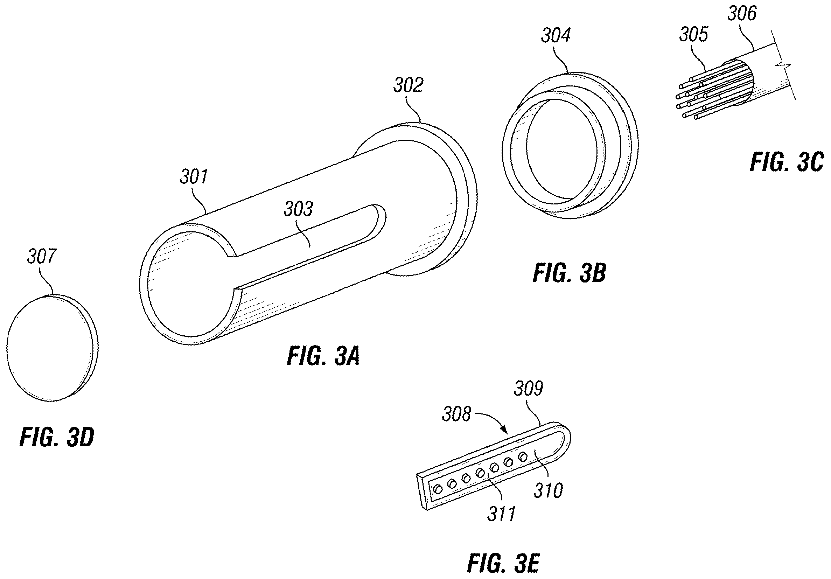

In contrast to many conventional IPGs, pulse generator 150 may comprise attached extension component 170. That is, in lieu of providing a separate extension lead that is physically placed within a header of an IPG by the surgeon during implant, extension component 170 may be directly attached to and may be non-removable from pulse generator 150 according to some representative embodiments. Although the integrated extension component 170 is provided for some embodiments, extension component 170 may be separate according to other embodiments. The welding techniques and components disclosed herein may be employed within any suitable implantable pulse generating system for applying pulses to tissue of a patient. Within pulse generator 150, electrical pulses are generated by pulse generating circuitry 152 and are provided to switching circuitry 157. The switching circuit connects to output wires, traces, lines, or the like (not shown in FIG. 3) which are, in turn, electrically coupled to internal conductive wires (not shown in FIG. 3) of lead body 302 of extension component 170. The conductive wires, in turn, are electrically coupled to electrical connectors (e.g., "Bal-Seal" connectors) within connector portion 171 of extension component 170. The terminals of one or more stimulation leads 110 are inserted within connector portion 171 for electrical connection with respective connectors. Thereby, the pulses originating from pulse generator 150 and conducted through the conductors of lead body 172 are provided to stimulation lead 110. The pulses are then conducted through the conductors of lead 110 and applied to tissue of a patient via electrodes 111. Any suitable known or later developed design may be employed for connector portion 171.

For implementation of the components within pulse generator 150, a processor and associated charge control circuitry for an implantable pulse generator is described in U.S. Patent Publication No. 20060259098, entitled "SYSTEMS AND METHODS FOR USE IN PULSE GENERATION," Circuitry for recharging a rechargeable battery of an implantable pulse generator using inductive coupling and external charging circuits are described in U.S. Patent Serial No. 11/109,114, entitled "IMPLANTABLE DEVICE AND SYSTEM FOR WIRELESS COMMUNICATION,"

An example and discussion of "constant current" pulse generating circuitry is provided in U.S. Patent Publication No. 20060170486 entitled "PULSE GENERATOR HAVING AN EFFICIENT FRACTIONAL VOLTAGE CONVERTER AND METHOD OF USE,". One or multiple sets of such circuitry may be provided within pulse generator 150. Different pulses on different electrodes may be generated using a single set of pulse generating circuitry using consecutively generated pulses according to a "multi-stimset program" as is known in the art. Alternatively, multiple sets of such circuitry may be employed to provide pulse patterns that include simultaneously generated and delivered stimulation pulses through various electrodes of one or more stimulation leads as is also known in the art. Various sets of parameters may define the pulse characteristics and pulse timing for the pulses applied to various electrodes as is known in the art. Although constant current pulse generating circuitry is contemplated for some embodiments, any other suitable type of pulse generating circuitry may be employed such as constant voltage pulse generating circuitry.

Stimulation lead(s) 110 may comprise a lead body of insulative material about a plurality of conductors within the material that extend from a proximal end of lead 110 to its distal end. The conductors electrically couple a plurality of electrodes 111 to a plurality of terminals (not shown) of lead 110. The terminals are adapted to receive electrical pulses and the electrodes 111 are adapted to apply stimulation pulses to tissue of the patient. Also, sensing of physiological signals may occur through electrodes 111, the conductors, and the terminals. Additionally or alternatively, various sensors (not shown) may be located near the distal end of stimulation lead 110 and electrically coupled to terminals through conductors within the lead body 172. Stimulation lead 110 may include any suitable number of electrodes 111, terminals, and internal conductors.

FIGS. 2A-2C respectively depict stimulation portions 200, 225, and 250 for inclusion at the distal end of lead 110. Stimulation portion 200 depicts a conventional stimulation portion of a "percutaneous" lead with multiple ring electrodes. Stimulation portion 225 depicts a stimulation portion including several "segmented electrodes." The term "segmented electrode" is distinguishable from the term "ring electrode." As used herein, the term "segmented electrode" refers to an electrode of a group of electrodes that are positioned at the same longitudinal location along the longitudinal axis of a lead and that are angularly positioned about the longitudinal axis so they do not overlap and are electrically isolated from one another. Example fabrication processes are disclosed in U.S. Provisional Patent Application Serial No. 61/247,360 , entitled, "METHOD OF FABRICATING STIMULATION LEAD FOR APPLYING ELECTRICAL STIMULATION TO TISSUE OF A PATIENT," . Stimulation portion 250 includes multiple planar electrodes on a paddle structure.

Although not required for all embodiments, the lead bodies of lead(s) 110 and extension component 170 may be fabricated to flex and elongate in response to patient movements upon implantation within the patient. By fabricating lead bodies according to some embodiments manner, a lead body or a portion thereof is capable of elastic elongation under relatively low stretching forces. Also, after removal of the stretching force, the lead body is capable of resuming its original length and profile. For example, the lead body may stretch 10%, 20%, 25%, 35%, or even up or above to 50% at forces of about 0.5, 1.0, and/or 2.0 pounds of stretching force.

The ability to elongate at relatively low forces may present one or more advantages for implantation in a patient. For example, as a patient changes posture (e.g., "bends" the patient's back), the distance from the implanted pulse generator to the stimulation target location changes. The lead body may elongate in response to such changes in posture without damaging the conductors of the lead body or disconnecting from pulse generator. Also, deep brain stimulation implants, cortical stimulation implants, and occipital subcutaneous stimulation implants usually involve tunneling of the lead body through tissue of the patient's neck to a location below the clavicle. Movement of the patient's neck subjects a stimulation lead to significant flexing and twisting which may damage the conductors of the lead body. Due to the ability to elastically elongate responsive to movement of the patient's neck, certain lead bodies according to some embodiments are better adapted for such implants than some other known lead body designs. Fabrication techniques and material characteristics for "body compliant" leads are disclosed in greater detail in U.S. Provisional Patent Application Serial No. 60/788,518, entitled "Lead Body Manufacturing," filed 03/31/2006.

Controller device 160 may be implemented to recharge battery 153 of pulse generator 150 (although a separate recharging device could alternatively be employed). A "wand" 165 may be electrically connected to controller device through suitable electrical connectors (not shown). The electrical connectors are electrically connected to coil 166 (the "primary" coil) at the distal end of wand 165 through respective wires (not shown). Typically, coil 166 is connected to the wires through capacitors (not shown). Also, in some embodiments, wand 165 may comprise one or more temperature sensors for use during charging operations.

The patient then places the primary coil 166 against the patient's body immediately above the secondary coil (not shown), i.e., the coil of the implantable medical device. Preferably, the primary coil 166 and the secondary coil are aligned in a coaxial manner by the patient for efficiency of the coupling between the primary and secondary coils. Controller 160 generates an AC-signal to drive current through coil 166 of wand 165. Assuming that primary coil 166 and secondary coil are suitably positioned relative to each other, the secondary coil is disposed within the field generated by the current driven through primary coil 166. Current is then induced in secondary coil. The current induced in the coil of the implantable pulse generator is rectified and regulated to recharge battery 153 by charging circuitry 154. Charging circuitry 154 may also communicate status messages to controller 160 during charging operations using pulse-loading or any other suitable technique. For example, controller 160 may communicate the coupling status, charging status, charge completion status, etc.

External controller device 160 is also a device that permits the operations of pulse generator 150 to be controlled by user after pulse generator 150 is implanted within a patient, although in alternative embodiments separate devices are employed for charging and programming. Also, multiple controller devices may be provided for different types of users (e.g., the patient or a clinician). Controller device 160 can be implemented by utilizing a suitable handheld processor-based system that possesses wireless communication capabilities. Software is typically stored in memory of controller device 160 to control the various operations of controller device 160. Also, the wireless communication functionality of controller device 160 can be integrated within the handheld device package or provided as a separate attachable device. The interface functionality of controller device 160 is implemented using suitable software code for interacting with the user and using the wireless communication capabilities to conduct communications with IPG 150.

Controller device 160 preferably provides one or more user interfaces to allow the user to operate pulse generator 150 according to one or more stimulation programs to treat the patient's disorder(s). Each stimulation program may include one or more sets of stimulation parameters including pulse amplitude, pulse width, pulse frequency or inter-pulse period, pulse repetition parameter (e.g., number of times for a given pulse to be repeated for respective stimset during execution of program), etc. IPG 150 modifies its internal parameters in response to the control signals from controller device 160 to vary the stimulation characteristics of stimulation pulses transmitted through stimulation lead 110 to the tissue of the patient. Neurostimulation systems, stimsets, and multi-stimset programs are discussed in PCT Publication No. WO 01/93953, entitled "NEUROMODULATION THERAPY SYSTEM," and US Patent No. 7,228,179, entitled "METHOD AND APPARATUS FOR PROVIDING COMPLEX TISSUE STIMULATION PATTERNS,". .

FIGS. 3A-3E depict respective components for creating an electrical connection from within the housing of a pulse generator to wire conductors of a lead body according to one representative embodiment.

FIG. 3A depicts tube 301. Although shown as a cylindrical structure in FIG. 3A, tube 301 may alternatively possess any other suitable cross-sectional shape (e.g., oval, rectangular, etc.). Tube 301 comprises slot 303 and, optionally, flange 302. FIG. 3B depicts end piece 304. FIG. 3D depicts cap structure 307. Tube 301, end piece 304, and cap 307 are preferably fabricated from the same type of material as employed for the material of the "can" or housing of pulse generator 150 (or, alternatively, a metallurgically compatible material). For example, suitable titanium materials or alloys may be employed for one or more of these components according to some embodiments. One or more of tube 301, end piece 304, and cap 307 may be fabricated using suitable metal processing techniques. For example, tube 301 may be fabricated using metal extrusion with post extrusion processing to create slot 303 and flange 302. Alternatively, metal injection molding may be employed for one or more of tube 301, end piece 304, and cap 307 depending upon the component complexity selected for a specific implementation.

FIG. 3B depicts lead body 306 that includes a plurality of conductor wires 305 within insulative material. At the proximal end of lead body, a length of each conductor wire 305 extends out from the insulative material. Lead body 306 may be fabricated using any known or later developed process. Examples of various lead body fabrication processes are disclosed in U.S. Patent No. 6,216,045, U.S. Patent No. 7,287,366, U.S. Patent Application Publication No. 20050027340A1, and U.S. Patent Application Publication No. 20070282411A1.

FIG. 3E depicts feedthrough assembly 308. Feedthrough assembly 308 comprises a plurality of feedthrough pins 310. Feedthrough pins 310 preferably extend through surface 311 from the "back" side of assembly 308 to the "front" side of assembly 308. In other embodiments, pins 310 need not extend through surface 311 and may be electrically coupled to one or more intermediate electrical components to extend the electrical connection through to the other side. Feedthrough assembly 300 also comprises ferrule 309 about the perimeter of assembly 300. Ferrule 309 is shaped to allow ferrule 309 to be attached to slot 303 and to allow the end of tube 301 to be sealed upon subsequent operations. Feedthrough assembly 308 may be fabricated using conventional techniques for feedthrough components, although feedthrough assembly 309 comprises a different structural design than conventional feedthrough components.

These various components are assembled and welded (e.g., using a suitable laser welding system) together to form an integrated structure before being coupled with the housing of pulse generator 150. In one embodiment, end piece 304 is placed over the insulative material of lead body 306. Then, tube 301 is likewise placed over the insulative material of lead body 306. With tube 301 placed sufficiently far along lead body 306 that it does not appreciable obstruct operations, the various wires 305 of lead body 306 are welded to respective feedthrough pins 310 (on the back side) of feedthrough assembly 308 (e.g., using the laser welding system or resistive welding). Non-conductive adhesive may also be applied to fix and reinforce the connection between wires 305 and pins 310. Tube 301 is slid back along lead body 306 such that assembly 308 is disposed in slot 303. Welding is applied to connect tube 301 to end piece 304 and to connect tube 301 to ferrule 309 of assembly 308 (preferably, using the laser welding system). In one specific embodiment, biocompatible polymer material (e.g., silicone or urethane materials) may be injected or otherwise provided within tube 301 before tube 301 is sealed through the welding to provide support for wires 305. End cap 307 is then welded to the distal end of tube 301 (preferably, using the laser welding system) to seal tube 301. A medical adhesive may also be applied where lead body 306 enters tube 301 to provide a non-hermetic seal.

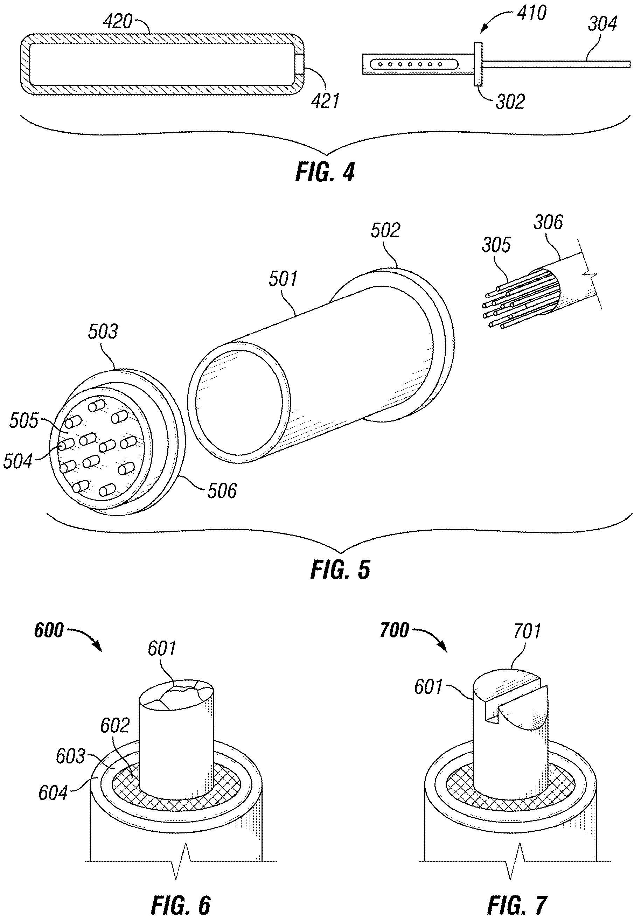

After performing the welding of these components, intermediate assembly 410 is formed (as shown in FIG. 4). Intermediate assembly 410 is then integrated with the housing component(s) 420 of pulse generator 150. Specifically, housing component(s) 420 may include an aperture 421 along one of its surfaces. Intermediate assembly 410 is placed through aperture 421 with flange 302 placed against the outer surface of housing components 420. Flange 302 is then welded to housing component(s) 420. Upon completion of the welding operations, the internal components of pulse generator 150 within housing component(s) 420 are hermetically sealed while being electrically connected to the conductive wires of lead body 306. Connector portion 171 may be provided at the proximal end of lead body 306 before or after intermediate assembly 410 is integrated with housing component(s) 420. Any suitable known or later developed technique for providing connector portion 171 may be employed.

In some embodiments, tube 301 is adapted to provide a frictional fit with lead body 306. Specifically, tube 301 may provide a sufficiently large frictional force to prevent lead body 306 from disengaging from the electrical connections formed with pins 310 feedthrough component 308 by stretching forces experienced in the patient's body after implantation. The interior surface of tube 301 may be adapted to contact lead body 306 for this purpose. The interior diameter of tube 301 may be sized to provide sufficient frictional contact. Also, crimping, swaging, or similar operations on tube 301 about lead body 306 may be employed to facilitate the desired frictional contact.

FIG. 5 depicts components for an intermediate assembly of components for an extension component for integration with a pulse generator housing according to another embodiment. As shown in FIG. 5, tube 501 is a hollow, substantially cylindrical structure, although any suitable cross-sectional shape may be employed. Tube 501 includes flange 502 at its distal end. Feedthrough assembly 503 includes a plurality of conductive pins disposed through ceramic or other suitable insulative material 505. Material 505 is surrounded by metallic material of ferrule 506. Tube 501 and feedthrough assembly 503 may be fabricated using the same materials and techniques as discussed above in regard to tube 301 and feedthrough assembly 309.

During assembly, tube 501 is initially slid over wire conductors 305 of lead body 306 until tube 501 is sufficiently advanced over lead body 306 so that it does not obstruct further operations. The various wire conductors 305 of lead body 306 are coupled to respective pins 504 of feedthrough assembly 503. Pins 504 may extend through material 511 or alternatively may partially extend while being connected to intermediate electrical components. Tube 501 is the slid into position so that tube 501 is set flush against feedthrough assembly 503. Feedthrough assembly 503 is welded to tube 501 (e.g., using a laser welding system) to form an intermediate assembly. Tube 501 is then preferably backfilled with suitable biocompatible material (e.g., silicone). The intermediate assembly is placed within housing component(s) of an implantable pulse generator and is welded to the housing components to hermetically seal the implantable pulse generator.

Conventional feedthrough pins are made using approximately 0.013 inch diameter solid platinum wire with a melting temperature of 1773° C. Conductor wires 305 for lead bodies 306 commonly include seven strands (48 gauge) of MP35N where six strands are served around one of the strands thereby resulting in a diameter of approximately 0.003 inches. MP35N has a melting temperature of 1440° C. The thermal diffusivity of MP35N is approximately 2.82° C · 10⁻⁶ M²/s and the thermal diffusivity of platinum is approximately 2.58° C · 10⁻⁵ M²/s, a difference of approximately a factor of 10. Additionally, the individual strands of MP35N can also have a silver core, which has a melting temperature of 963° C. Further, the reflectivity of these materials to wavelengths used by laser welding systems differs. These differences in reflectivity, melting temperature, thermal diffusivity, and diameter of platinum and MP35 stranded wires contribute to the complexity of attaching wires 305 to pins 310 using laser welding.

Specifically, during a welding pulse, sufficient energy is presented to each material to create a melt zone in the metals so the metals will join together and solidify into the same mass and produce a metallurgical bond. Since platinum wire used for pins 310 is much larger and has a much greater melt temperature, the platinum wire for pins 310 requires more energy to melt than wires 305. If this amount of energy is presented to the one wire 305 positioned on the surface of a corresponding platinum pin 310 such that the laser impinges directly on the MP35N material, the wire 305 can pull away from the platinum material of pin 310 and no bond will result.

This may be caused by the fine strands of MP35N melting and the surface tension of the MP35N/silver molten material "balling up" and forming a spheroid shape thereby pulling away form the platinum material of pin 310. The process may take place before the platinum melts thereby preventing a bond from occurring. If this occurs, it is possible that the exposed portion of wire 305 may be too short to reach the platinum pin 310. If there is a long enough service loop of MP35N wire, the wire may be repositioned and another weld may be attempted. However, provision of a suitable service loop may be impossible or impractical to employ for small, precision assemblies.

In some embodiments, one or more adaptations are provided to facilitate electrical connection of wires 305 of lead body 306 to pins 310. FIGS. 6 and 7 depict an adaptation according to one representative embodiment. FIG. 6 depicts feedthrough component 600. Feedthrough component 600 comprises platinum pin 601. Pin 601 is surrounded by gold 602 and ceramic material 603. Ferrule 604 is applied around ceramic material 603. In the state shown in FIG. 6, feedthrough component 600 may be fabricated using conventional brazing techniques. After further processing, feedthrough component 700 is provided as shown in FIG. 7. In FIG. 7, pin 601 of feedthrough component 700 comprises laser machined slot 701. Alternatively, other surface feature designs could be provided such as an "X" or cruciform-type surface feature. In one embodiment, pin 601 is initially melted using an infrared (IR) laser. While in its molten state, an ultraviolet (UV) laser is employed to laser machine pin 601 to obtain the desired surface profile and surface feature(s).

By employing the surface feature, wire 305 may be placed in slot 701 during a laser welding operation. The laser energy may be readily provided to pin 601 to melt pin 601 without inadvertently causing wire 305 to pull away before the welding operation is completed. That is, the laser energy will impinge upon pin 601 thereby melting pin 601. Wire 305 will largely be shielded from direct exposure to the laser energy by its position within slot 701 and will melt by conduction of heat from pin 601. In an alternative embodiment, a relatively lower amount of laser energy (e.g., less than the amount applied to pin 601) may be directly applied to wire 305 during the welding operation to directly heat wire 305 in addition to conductive heating.

Although only one pin 601 is shown in FIGS. 6 and 7, any suitable number of pins may be processed for a respective device. Also, the pins may be disposed in any suitable arrangement or array within a feedthrough assembly. Preferably, each pin in the feedthrough assembly is processed as discussed in regard to FIG. 7. Each slot in the pins of the feedthrough assembly may have the same orientation. Alternatively, selected slots may be oriented or "clocked" differently depending upon any constraints created by a given design of the lead body, the feedthrough assembly, housing components, or other components.

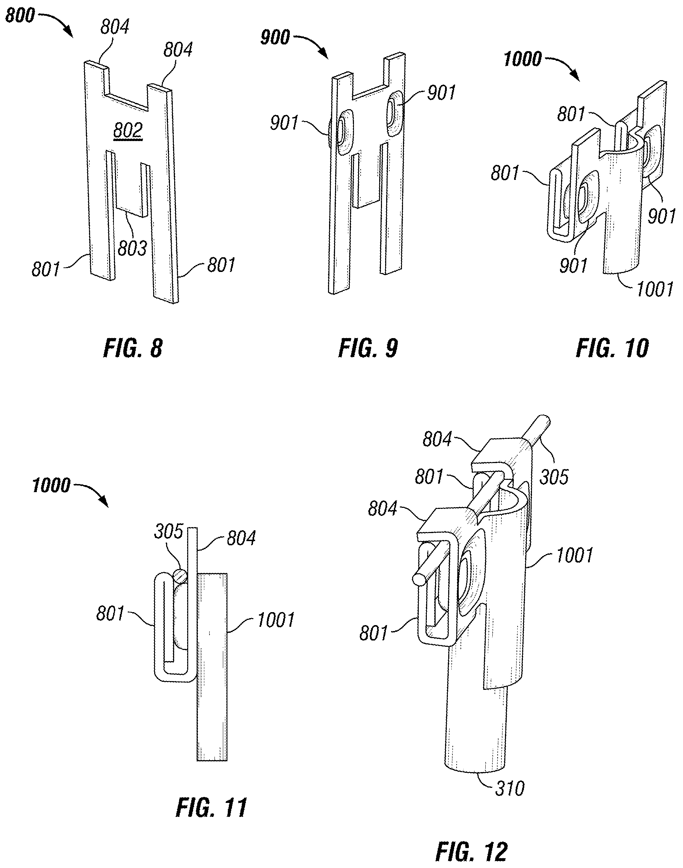

FIGS. 8-12 depict a series of structures (during various processing steps) during connection of wire 305 to pin 310 according to one representative embodiment. Selected steps shown in these FIGS. may employ conventional forming or stamping methods (e.g., using a four slide or multi-slide forming tool). In one embodiment, as shown in FIG. 8, flat platinum, platinum iridium, MP35N, or other alloy ribbon stock 800 is formed using any suitable metal forming and/or processing technique(s). Ribbon stock 800 comprises two lateral longitudinal members 801 which are joined by medial portion 802. Ribbon stock 800 further comprises central extension member 803 which extends away from medial portion 802. Also, tab members 804 on longitudinal members 801 extend above medial portion 802. In one embodiment, dimples 901 are formed in the flat stock 800 to form intermediate component 900 as shown in FIG. 9.

As shown in FIG. 10, the flat stock is bent upon itself to form welding component 1000. Medial member and central extension member 803 are also bent to form a curved portion 1001. The concave surface of curved portion 1001 is adapted to be placed against a feedthrough pin during subsequent operations. Extension members 801 are folded over themselves to be coplanar with the face of dimples 901.

FIGS. 11 and 12 depict respective operations performed while joining wire 305 to feedthrough pin 310 (shown in FIG. 12) according to one representative embodiment.

Conductor wire 305 is placed between gap between folded longitudinal members 802 and tabs 804 as shown in FIG. 11. The size of the gap is selected to accommodate the size of wire 305 and is controlled, during the processing steps, by the depth of dimples 901. Tabs 804 are bent over wire 305 as shown in FIG. 12 to produce intimate contact between wire 305 with the surface of welding component 1000 and to retain the wire in the gap. In one embodiment, tabs 804 may apply a compressive force to wires 305 after tabs 804 are placed in position. Also, tabs 804 tend to manage the individual strands of wire 305 which may have a tendency to separate from the bundle.

In one embodiment, tabs 804 are adapted to shield wire 305 from direct exposure to the laser or resistance welder. The metal of tabs 804 may be directly heated into a molten state. For a laser weld, the laser energy may be directly focused on tabs 804 without directly impinging upon wire 305. Also, if resistance welding is employed to connect wire 305 to one or more tabs 804 of welding component 1000, the electrodes of the welder may be placed against tabs 804. Wire 305 may be heated to its melt temperature indirectly by conduction of heat from one or more of tabs 804. In this manner, wire 305 will tend to avoid pulling away and forming a spheroid mass during welding operations. Further, the tab geometry, size, thickness, and mass may be optimized for welding to wire 305. That is, the difference in energy between melting material of a respective tab 804 and melting material of wire 305 will be lessened thereby reducing the occurrence of wire 305 pulling away during a weld operation. Further, the mass per unit length of tabs 804 is relatively close (as compared to pin 310) to the mass per unit length of wire 305 which further assists successful completion of the welding process. In an alternative embodiment, a smaller amount of laser energy may be applied to wire 305 during the welding operation to directly heat wire 305 in addition to conductive heating.

During connection operations, the presence of two or more tabs 804 may enable a greater manufacturing yield. Specifically, a welding attempt preferably occurs on the tab 804 at the distal most portion of wire 305. In the event that the initial weld operation is not optimal, another weld attempt may be made on the other or next adjacent tab 804 where the wire 305 is unaffected by the first weld attempt.

The connection of welding component 1000 to pin 310 may occur independently of the connection of wire 305 to welding component 1000. Welding component 1000 may be connected to pin 310 first or wire 305 may be connected first at any suitable stage in the overall manufacturing process. As shown in FIG. 12, welding component 1000 may be placed against a respective feedthrough pin 310. Surface 1001 is preferably adapted to conform to the diameter of pin 310. Welding component 1000 may then be connected to the feedthrough pin 310 using any suitable technique including resistive welding and laser welding.

In some embodiments, welding component 1000 further enables tabs 804 to be "clocked" in any suitable orientation to aid guiding wire 305 into the gap between the tabs 804. To reduce the overall package size, the ability to orient tabs 804 in this manner aids in management of the wires 305, because the various wires 305 of lead body 306 will emanate from lead body 306 at different angles.

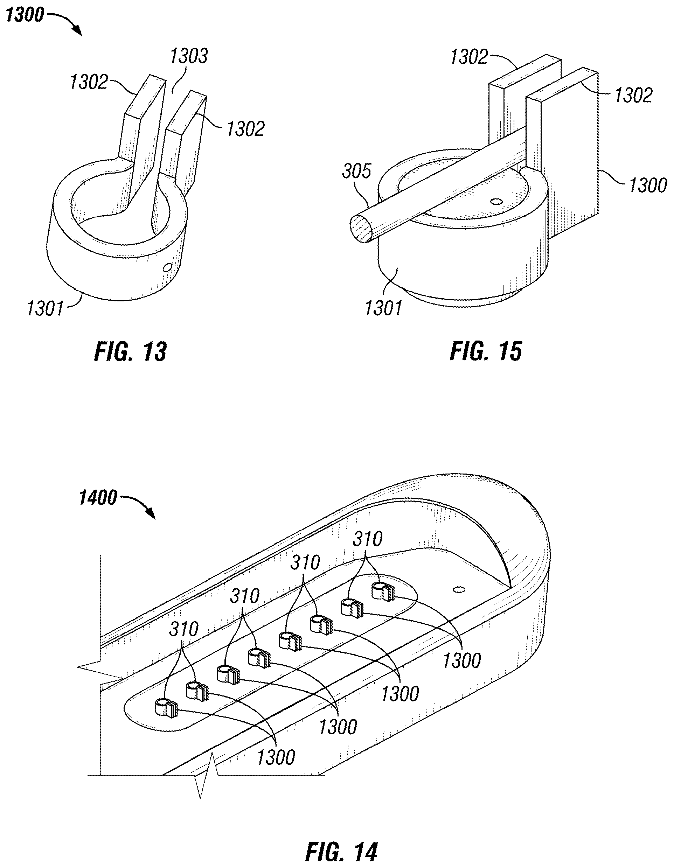

FIGS. 13-15 depict electrical connection of a respective wire 305 to a feedthrough pin 310 according to another representative embodiment. As shown in FIG. 13, ribbon clamp component 1300 is formed using conventional forming or stamping methods (e.g., using a four slide or multi-slide forming tool). Ribbon clamp component 1300 may be formed from platinum, platinum iridium, MP35N, or any other suitable alloy. Ribbon clamp component 1300 comprises curved portion 1301. Ribbon clamp component 1300 further comprises flat tab portions 1302 connected to and integral with curved portion 1301. Flat tab portions 1302 extend outward in an approximately radial direction. Also, flat tab portions 1302 are disposed adjacent to each other with gap 1303 provided between their interior surfaces. Gap 1303 is preferably sized to fit passage of wire 305 between flat tab portions 1302.

In use, a plurality of ribbon clamp components 1300 may be placed about respective feedthrough pins 310 as shown in FIG. 14. Preferably, the inner diameter of curved portion 1301 is selected according to the outer diameter of feedthrough pins 310 to facilitate this step. The band shape of curved portion 1301 facilitates locating ribbon clamp components 1300 on pins 310 and assists holding them in place so components 1300 function in a self-fixturing manner. Flat tab portions 1302 may be placed in any suitable orientation about pin axis to account for different wire angles from lead body 306 which assists assembly in reduced package-design devices. Each ribbon clamp component 1300 is preferably welded to its respective pin 310 (e.g., using laser welding or resistive welding).

A respective wire 305 is also placed within gap 1303 between flat tab portions 1302 of each ribbon clamp component 1300. After placement of the wire 305, flat tab portions 1302 may be bent around and against the wire 305 to produce intimate contact and to retain wire 305 in gap 1303. Also, flat tab portions 1302 assist in managing the individual strands of wire 305 which may have a tendency to separate from the bundle. Preferably, at this point, flat tab portions 1302 apply a compressive force to wire 305.

In one embodiment, flat tab portions 1302 are adapted to shield wire 305 from direct exposure to the laser or resistance welder. The metal of flat tab portions 1302 may be directly heated into a molten state. For a laser weld, the laser energy may be directly focused on flat tab portions 1302 without directly impinging upon wire 305. Also, if resistance welding is employed to connect wire 305 to flat tab portions 1302 of welding component 1300, the electrodes of the welder may be placed again flat tab portions 1302. Wire 305 may be heated to its melt temperature indirectly by conduction of heat from flat tab portions 1302. In this manner, wire 305 will tend to avoid pulling away and forming a spheroid mass during welding operations. Further, the tab geometry, size, thickness, and mass may be optimized for welding to wire 305. That is, the difference in energy between melting material of flat tab portions 1302 and melting material of wire 305 will be lessened thereby reducing the occurrence of wire 305 pulling away during a weld operation. Further, the mass per unit length of flat tab portions 1302 is relatively close (as compared to pin 310) to the mass per unit length of wire 305 which further assists successful completion of the welding process.

The welding of a respective component 1300 to a corresponding pin 310 and welding of the component 1300 to a corresponding wire 305 may occur in the same step. Alternatively, the welding of these elements may occur at separate times during the overall device fabrication process.

In other embodiments, welding components 1000 and 1300 providing additional geometry for reworking bad or failed weld joints. Specifically, the more complex geometry of weld components 1000 and 1300 as compared to the cylindrical shape of the feedthrough pin provides additional locations for further weld attempts to bond the wire to the feedthrough pin after an unsuccessful initial weld operation.

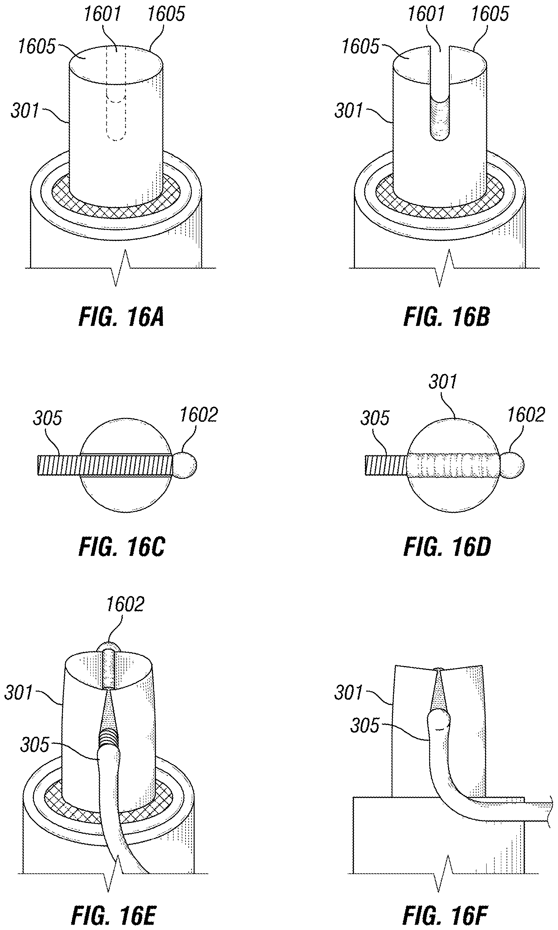

FIGS. 16A-16F depict processing of feedthrough pin 310 during creation of an electrical connection with conductor wire 305 for a pulse generator according to one representative embodiment. The process described for FIGS. 16A-16F is similar to the process discussed for FIGS. 6 and 7 with modifications. Any of the techniques discussed above may also be employed for the described below for FIGS. 16A-16F. In FIG. 16A, conventional brazed feedthrough pin 310 is shown. The pin 310 may be cut to a defined length using a UV laser. The laser cutting operation also preferably creates a flat surface on pin 310. Notch 1601 in pin 310 is made along an axis to accommodate a conductor wire as shown in FIG. 16B (e.g., using the same UV laser). Posts 1605 are defined on either side of notch 1601 by the removal of the material from pin 310 during the laser machining operation. In one embodiment, notch 1601 is formed slightly wider (e.g., 0.001 inches wider) than the outer diameter of the conductor wire to be connected to pin 310. In one embodiment, the depth of notch 1601 is approximately twice the outer diameter of the conductor wire to facilitate subsequent operations.

At a suitable time, preferably prior to placement of conductor 305 within notch 1601, the end of stranded conductor wire 305 is melted using a YAG laser welding system. This welding operation is performed using a sufficiently large laser beam to include the full diameter of wire 305. This welding operation forms a single ball of metal at the end of wire 305 from the strands of the DFT wire. The wire 305 is placed within notch 1601 of pin 310 with ball 1602 disposed outside the end of notch 1601 and adjacent to the exterior surface of pin 310 as shown in FIG. 16C. Posts 1605 are preferably then pinched together to clasp about wire 305. The pinching operation preferably applies a compressive force to wire 305. The distal edges of posts 1605 above wire 305 are brought into contact with each other as shown in FIG. 16D.

Using a YAG laser welding system, posts 1605 are seam welded together as also shown in FIG. 16D. FIGS. 16E and 16F depict different perspective views of pin 310 with wire 305 after the seam welding. The laser path preferably extends from just beyond the edge of posts 1065 on the pre-formed ball 1602 of wire 305. As the seam weld progresses, ball 1062 and pin 310 will bond making an electrical contact. Also, the seam will secure posts 1605 about the portion of wire 305 subject to a compressive force by posts 1605. Wire 305 is thereby held in place by pinched posts 1605 and the laser weld formed by ball 1062 at the end of wire 305 with pin 310. The combination of these characteristics provide a robust electrical contact and a securely held wire 305.

Alternative laser systems may be employed for any of the embodiments discussed herein. For example, a picosecond or femtosecond laser system may be employed to cut and machine pin 310 using extremely short pulses. In lieu of a YAG laser system, a fiber laser may be employed using IR wavelengths. Also, although certain discussions have include example order of processing steps, any suitable order of processing and assembly of the various components during pulse generator fabrication may be employed according to some embodiments.

In some embodiments, a ferrule tube connector may be employed to electrically connect a feedthrough pin 310 to a conductor wire. The tube connector is preferably adapted to be self-fixturing and to position and hold a conductor wire in place for the welding process. The ferrule tube connector may be fabricated from platinum, a platinum alloy, or any other suitable conductive material. The tubular shape may be obtained by machining or extruding as examples. In one embodiment shown, the ferrule tube connector is provided which includes an inner diameter that is approximately sized according to the outside diameter of pin 310 to obtain an intimate fit up between the ferrule tube connector and pin 310. During fabrication operations, the tube connector is placed circumferentially about pin 310. The tube connector is preferably adapted to provide an optimal mass for forming a weld joint between feedthrough pin 310, the tube connector, and wire 305. Suitable operations discussed in regard to FIGS. 16A-16F may also be employed to electrical connect these components.

In some embodiments, the ferrule tube connector further comprises a slot defined across the upper surface of the connector. The slot may be sized to only allow wire 305 to pass through the slot across the connector in a longitudinal manner. Alternatively, the slot may be sized in a wider fashion to allow a laser spot to be focused on pin 310 and a combination of pin 310 and the tube connector (or even slight impinging upon wire 305 also). In one embodiment, the slot has a width of approximately 0.018 inches. During fabrication, wire 305 is placed in the slot. Wire 305 may be placed across the entire length of slot. Alternatively, wire 305 may be placed on one side of the slot to maintain exposure of pin 310 to application of the laser spot during welding operations. Also, the ferrule tube connector may optionally be slightly crimped about wire 305 before wire 305 is welded to the tube connector.

In some embodiments, a single weld operation occurs to weld the tube connector, pin 310, and wire 305. The laser energy may be largely directed on the tube connector and/or pin 310 thereby improving the probability of obtaining a robust weld between wire 305 and tube connector 1700. Other variations in the welding order are possible. For example, the tube connector may be initially welded to pin 310 before pin 310 is integrated with the hermetic housing of the implantable medical device. A separate weld operation may be performed later the connect wire 305 to the tube connector.

In another embodiment, wire 305 is first welded to the tube connector to form a separate sub-assembly and then this sub-assembly is mated and welded with the feedthrough pin 310. This embodiment may involve (1) reduced operator interaction with the IPG feed through, (2) completion of portions of the header assembly before being attachment to the IPG, (3) reduction of IPG yield losses due to poor wire welds as compared to selected known manufacturing processes.

## Claims

1. A method of fabricating an implantable pulse generator for generating electrical pulses for application to tissue of a patient, the method comprising:
providing one or more housing components adapted to house at least pulse generating and switching circuitry of the implantable pulse generator;
providing a feedthrough assembly adapted to be coupled with the one or more housing components, the feedthrough assembly comprising an array of feedthrough pins for providing respective electrical connections from a hermetically sealed enclosure formed within the one or more housing components to a location exterior to the enclosure;
providing a plurality of welding components with each welding component comprising a first portion adapted to engage a respective feedthrough pin and a second portion for engaging a conductor wire;
performing one or more weld operations for each welding component to connect each welding component with a respective feedthrough pin and a respective conductor wire by applying a majority of welding energy directly to the welding components;
wherein, within the enclosure, each feedthrough pin is electrically coupled to the pulse generating circuitry through the switching circuitry of the implantable pulse generator;
**characterised in that**
wherein (i) each of the plurality of welding components is ferrule tube connector, (ii) each of the feedthrough pins are welded to a respective ferrule tube connector that circumscribes the corresponding feedthrough pin and (iii) each of the plurality of conductor wires is disposed within a slot of a corresponding ferrule tube connector with the respective conductor wire being welded to its corresponding ferrule tube connector.

2. The method of claim 1, further comprising:
applying laser energy to each of the plurality of conductors to melt strands of each conductor to form a ball structure at a distal end of the second portion.

3. The method of claim 2, wherein the ball structure at the distal end of each conductor comprises a diameter greater than a width of the slots formed in ferrule tube connectors and, optionally,
the placing comprises:
placing each conductor within the slot of its corresponding ferrule tube connector with its ball structure disposed against an exterior surface of its corresponding ferrule tube connector.

4. The method of claim 1, further comprising:
pinching portions of each ferrule tube connector about the slot of the respective ferrule tube connector to bend the portions to clasp about its corresponding conductor and, optionally,
the performing welding operations comprises seam welding the bent portions of each ferrule tube connector about its corresponding conductor.

5. The method of claim 1, wherein portions of the conductor wires that are distal to the welding components are enclosed by insulative material of a lead body and, optionally,
the lead body is coupled to a connector component adapted to electrically connect to terminals of a stimulation lead.

6. The method of claim 1, further comprising:
bending one or more tabs of each welding component to secure its corresponding conductor wire in place before welding the conductor wire to the respective welding component, or
the performing one or more weld operations comprises: applying laser energy to the welding component wherein the welding component comprises one or more tabs adapted to shield the corresponding conductor wire of the welding component from direct exposure to the applied laser shield, or
the performing one or more weld operations comprises: contacting electrodes of a resistance welding system against tabs of a welding component without directly contacting the corresponding conductor wire of the welding component.

7. The method of claim 1, further comprising:
adjusting an orientation of tabs of one of the welding components according to an orientation of its corresponding wire.

8. The method of claim 1, wherein the performing one or more weld operations comprises:
performing a first weld operation to weld the welding component to its corresponding feedthrough pin;
performing a second weld operation to weld the welding component to its corresponding conductor wire, or
welding component is welded to its corresponding feedthrough pin and its corresponding conductor wire in a sub-stantially simultaneous manner, or
the performing one or more weld operations causes each conductor wire to melt by conduction of heat from its corresponding welding component.

9. The method of claim 1, wherein each welding component comprises a curved surface adapted to mate with a surface of each feedthrough pin.

10. The method of claim 9, wherein each welding component comprises:
an indent adjacent to a tab structure for receiving a conductor wire and, optionally,
the performing one or more weld operations comprises:
attempting a first weld operation on a first tab of one of the weld components to weld to a corresponding conductor wire at an end portion of the conductor wire; and
performing a second weld operation, upon unsuccessful completion of the first weld operation, on a second tab of the one of the weld components to weld to the corresponding conductor wire at a relatively medial portion of the conductor wire.

11. An implantable pulse generator, comprising:
a housing for hermetically enclosing pulse generating circuitry and switching circuitry of the implantable pulse generator;
a feedthrough assembly welded to the housing, the feedthrough assembly comprising a plurality of feedthrough pins extended from within an interior space within the housing to a location exterior to the housing, the feedthrough pins being electrically coupled to the pulse generating circuitry through the switching circuitry;
an extension component comprising (i) a lead body extending away from the housing, wherein the lead body encloses a plurality of conductor wires enclosed within insulative material, and
a plurality of welding components that each comprise a first portion welded to a corresponding feedthrough pin of the feedthrough assembly **characterised in that** each of the plurality of welding components comprises at least one tab structure at least partially surrounding and welded to a corresponding conductor wire of the lead body; and **in that** the extension component comprises (ii) an electrical connector portion adapted to receive terminals of a stimulation lead, wherein the extension component is non-removably attached to the housing.

12. The implantable pulse generator of claim 11, wherein the first portion of each welding component comprises a curved surface corresponding to an outside diameter of the plurality of feedthrough pins.

13. The implantable pulse generator of claim 11, wherein each welding component comprises two tabs disposed on opposite sides of the corresponding conductor wire of the respective welding component and, optionally,
each welding component comprises an indent holding the corresponding conductor wire of the respective welding component and the at least one tab structure is bent over the corresponding conductor wire.

14. The implantable pulse generator of claim 11, wherein each welding component comprises two tabs extending in a sub-stantially radial direction away from the feedthrough pin.

15. The implantable pulse generator of claim 11, wherein at least some of the plurality of welding components are oriented in different directions on the feedthrough pins of the feedthrough assembly.

## Patentansprüche

1. Verfahren zum Herstellen eines implantierbaren Pulsgenerators zum Generieren elektrischer Pulse für die Anwendung an Gewebe eines Patienten, wobei das Verfahren umfasst:
Bereitstellen eines oder mehrerer Gehäusekomponenten, welche dazu geeignet sind, mindestens eine Puls generierende und eine schaltende Schaltung eines implantierbaren Generators aufzunehmen;
Bereitstellen einer Zuführungsanordnung, der geeignet ist, mit der einen oder den mehreren Gehäuseelementen verbunden zu werden, wobei die Zuführungsanordnung eine Anordnung aus Durchführungsstiften zum Bereitstellen von entsprechenden elektrischen Verbindungen von einer hermetisch abgedichteten Einschließung, welche innerhalb der einen oder den mehreren Gehäusekomponenten zu einem Ort außerhalb der Einschließung ausgebildet ist;
Bereitstellen einer Vielzahl von Schweißkomponenten, wobei jede Schweißkomponente ein erstes Teil umfasst, welches geeignet ist, mit einem entsprechenden Durchführungsstift in Eingriff zu kommen, und ein zweites Teil für den Eingriff mit einem Leitungsdraht umfasst;
Durchführen einer oder mehrerer Schweißoperationen für jede Schweißkomponente, um jede Schweißkomponente mit einem entsprechenden Durchführungsstift und einem entsprechenden Leitungsdraht durch Aufbringen einer Vielzahl an Schweißenergie direkt an den Schweißkomponenten zu verbinden;
wobei innerhalb der Einschließung jeder Durchführungsstift elektrisch über die schaltende Schaltung des implantierbarer Pulsgenerators mit der Puls generierenden Schaltung gekoppelt ist, **dadurch gekennzeichnet, dass** (i) jede der Vielzahl an Schweißverbindungen eine hülsenartige Schlauchverbindung ist, dass (ii) jeder Durchführungsstift an einer entsprechenden hülsenartigen Schlauchverbindung geschweißt ist, welche den korrespondierenden Durchführungsstift abgrenzt,
und dass (iii) jeder der Vielzahl an Verbindungsdrähten in einem Schlitz eines entsprechenden hülsenartigen Schlauchverbinders angeordnet ist, wobei der entsprechende Verbindungsdraht an dem korrespondierenden hülsenartigen Schlauchverbinder angeschweißt ist.

2. Verfahren nach Anspruch 1, welches weiter umfasst:
Applizieren von Laserenergie an jedem der Vielzahl von Leitern, um den Draht eines jeden Leiters zur Bildung einer Kugelstruktur an einem distalen Ende des zweiten Teils zu schmelzen.

3. Verfahren nach Anspruch 2, wobei die Kugelstruktur an dem distalen Ende eines jeden Leiters einen Durchmesser aufweist, der größer als eine Breite des Schlitzes ist, welcher in der hülsenartigen Schlauchverbindung geformt ist, und wobei das Platzieren optional umfasst:
Platzieren eines jeden Leiters mit seiner Kugelstruktur innerhalb des Schlitzes seines entsprechenden hülsenartigen Schlauchverbinders, wobei die Kugelstruktur gegen eine äußere Fläche seines entsprechenden hülsenartigen Schlauchverbinders angeordnet ist.

4. Verfahren nach Anspruch 1, welches weiter umfasst:
Klemmabschnitte eines jeden hülsenartigen Schlauchverbinders entlang dem Schlitz eines entsprechenden hülsenartigen Schlauchverbinders zum Biegen der Teile, um diese an ihren entsprechenden Leiter fest zu klammern, und optional dass das Durchführen von Schweißoperationen ein Nahtschweißen der gebogenen Teile eines jeden hülsenartigen Schlauchverbinders über seinen entsprechenden Leiter umfasst.

5. Verfahren nach Anspruch 1, wobei Teile der Leitungsdrähte, welche distal zu den Schweißkomponenten angeordnet sind, von einem nicht leitenden Material eines Leitungskörpers umschlossen sind, optional, dass der Leitungskörper mit einer Verbindungskomponente verbunden ist, welche geeignet ist, elektrisch mit Anschlüssen einer Stimulationsleitung verbunden zu werden.

6. Verfahren nach Anspruch 1, welches weiter umfasst:
Biegen einer oder mehrerer Schlaufen einer jeden Schweißkomponente, um sie an ihrem entsprechenden Leitungsdraht an einer Stelle zu sichern, bevor der Leitungsdraht an die entsprechende Schweißkomponente geschweißt wird, oder das Durchführen einer oder mehrerer Schweißoperationen umfasst:
Applizieren von Laserenergie an die Schweißkomponente, wobei die Schweißkomponente eine oder mehrere Schlaufen umfasst, welche geeignet ist bzw. sind, den entsprechenden Leitungsdraht der Schweißkomponente von der direkten Einwirkung der applizierten Laserabschirmung zu schützen, oder das Durchführen einer oder mehrerer Schweißoperationen umfasst:
Zusammenziehen von Elektroden eines Widerstandschweißsystems gegen die Schlaufen einer Schweißkomponente, ohne den entsprechenden Leitungsdraht der Schweißkomponente direkt zu kontaktieren.

7. Verfahren nach Anspruch 1, welches weiter umfasst:
Einstellen einer Orientierung der Schlaufen einer der Schweißkomponenten entsprechend zu der Orientierung des zugehörigen Drahtes.

8. Verfahren nach Anspruch 1, wobei das Durchführen einer oder mehrerer Schweißoperationen umfasst:
Durchführen einer ersten Schweißoperation zum Anschweißen der Schweißkomponente an ihren zugehörigen Durchführungsstift;
Durchführen einer zweiten Schweißoperation zum Anschweißen der Schweißkomponente an ihren zugehörigen Leitungsdraht, oder
die Schweißkomponente wird an ihren zugehörigen Durchführungsstift und ihren zugehörigen Leitungsdraht im Wesentlichen gleichzeitig angeschweißt, oder
das Durchführen einer oder mehrerer Schweißoperationen verursacht, dass jeder Leitungsdraht durch die Wärmeleitung seiner zugehörige Schweißkomponente geschmolzen wird.

9. Verfahren nach Anspruch 1, wobei jede Schweißkomponente eine kurvige Fläche umfasst, welche geeignet ist, mit einer Oberfläche eines jeden Durchführungsstiftes zusammen zu passen.

10. Verfahren nach Anspruch 9, wobei jede Schweißkomponente umfasst:
eine Einkerbung neben einer Schlaufenstruktur, und optional, dass das Durchführen einer oder mehrerer Schweißoperationen umfasst:
einen Versuch einer ersten Schweißoperation an einer ersten Schlaufe einer der Schweißkomponenten an den entsprechenden Leitungsdraht an einem Endteil des Leitungsdrahtes anzuschweißen; und Durchführen einer zweiten Schweißoperation auf Basis einer nicht erfolgreich durchgeführten Fertigstellung einer ersten Schweißoperation an einer zweiten Schlaufe einer der Schweißkomponenten, um diese an den entsprechenden Leitungsdraht an einem relativ mittleren Teil des Leitungsdrahtes anzuschweißen.

11. Implantierbarer Pulsgenerator umfassend:
ein Gehäuse zum hermetischen Einschließen einer Puls generierenden Schaltung und einer schaltbaren Schaltung des implantierbaren Pulsgenerators;
eine an das Gehäuse geschweißte Zuführungsanordnung, wobei die Zuführungsanordnung eine Vielzahl an Durchführungsstiften umfasst, welche sich von einem Innenraum innerhalb des Gehäuses zu einer Stelle außerhalb des Gehäuses erstrecken, wobei die Durchführungsstifte an die Puls generierende Schaltung durch die schaltbare Schaltung elektrisch gekoppelt ist;
eine Erweiterungskomponente, welche (i) einen Führungskörper umfasst, welcher sich von dem Gehäuse erstreckt, wobei der Führungskörper eine Vielzahl an Leitungsdrähten umschließt, welche von einem nicht leitenden Material umschlossen sind, und eine Vielzahl an Schweißkomponenten, wobei jede Schweißkomponente einen ersten Teil, der an einen entsprechenden Durchführungsstift der Zuführungsanordnung geschweißt ist, umfasst, **dadurch gekennzeichnet** das jeder der Vielzahl an Schweißkomponenten mindestens eine Schlaufenstruktur, welche mindestens teilweise einen entsprechende Leitungsdraht des Leitungskörpers umgibt und an diesen angeschweißt ist, umfasst; und dass die Erweiterungskomponente (ii) einen elektrischen Verbindungsteil umfasst, der dazu geeignet ist, Anschlüsse eines Stimulationskörpers zu empfangen, wobei die Erweiterungskomponente nicht lösbar an dem Gehäuse angebracht ist.

12. Implantierbarer Pulsgenerator nach Anspruch 11, wobei der erste Teil einer jeden Schweißkomponente eine kurvige Fläche umfasst, welche einem äußeren Durchmesser der Vielzahl an Durchführungsstifte entspricht.

13. Implantierbarer Pulsgenerator nach Anspruch 11, wobei jede Schweißkomponente zwei Schlaufen aufweist, welche an gegenüberliegenden Seiten des entsprechenden Leitungsdrahtes der jeweiligen Schweißkomponente angeordnet sind, und optional wobei jede Schweißkomponente eine Einkerbung umfasst, welche den entsprechenden Leitungsdraht der zugehörigen Schweißkomponente hält und die mindestens eine Schlaufenstruktur über den zugehörigen Leitungsdraht gebogen ist.

14. Implantierbarer Pulsgenerator nach Anspruch 11, wobei jede Schweißkomponente zwei Schlaufen umfasst, welche sich in einer im Wesentlichen radialen Richtung von dem Durchführungsstift erstrecken.

15. Implantierbarer Pulsgenerator nach Anspruch 11, wobei mindestens einige der Vielzahl an Schweißkomponenten in verschiedenen Richtungen an den Führungsstiften der Führungsanordnung orientiert sind.

## Revendications

1. Procédé de fabrication d'un générateur d'impulsions implantable pour générer des impulsions électriques pour l'application à un tissu d'un patient, le procédé comprenant :
fournir un ou plusieurs éléments de logement agencés pour loger au moins des circuiteries de génération et de commutation d'impulsions du générateur d'impulsions implantable ;
fournir un ensemble traversée agencé pour être couplé au ou aux éléments de logement, l'ensemble traversée comprenant un réseau de broches de traversée pour fournir des raccordements électriques respectifs d'une enveloppe scellée hermétiquement, formée à l'intérieur du ou des éléments de logement, à un emplacement extérieur à l'enveloppe ;
fournir une pluralité d'éléments de soudage, chaque élément de soudage comprenant une première partie agencée pour engager une broche de traversée respective et une seconde partie pour engager un fil conducteur ;
réaliser une ou plusieurs opérations de soudage pour chaque élément de soudage pour relier chaque élément de soudage à une broche de traversée respective et un fil de conducteur respectif par application d'une majorité d'énergie de soudage directement aux éléments de soudage ;
dans lequel, à l'intérieur de l'enveloppe, chaque broche de traversée est couplée électriquement à la circuiterie de génération d'impulsions par l'intermédiaire de la circuiterie de commutation du générateur d'impulsions implantable ;
**caractérisé par le fait que**
(i) chacun de la pluralité d'éléments de soudage est un raccord tubulaire à ferrule, (ii) chacune des broches de traversée est soudée à un raccord tubulaire à ferrule qui entoure la broche de traversée correspondante, et (iii) chacun de la pluralité de fils conducteurs est disposé à l'intérieur d'une fente d'un raccord tubulaire à ferrule correspondant avec le fil conducteur respectif soudé à son raccord tubulaire à ferrule correspondant.

2. Procédé selon la revendication 1, comprenant en outre :
appliquer de l'énergie laser à chacun de la pluralité de conducteurs pour faire fondre des brins de chaque conducteur pour former une structure de bille à une extrémité distale de la seconde partie.

3. Procédé selon la revendication 2, dans lequel la structure de bille à l'extrémité distale de chaque conducteur comprend un diamètre plus grand qu'une largeur des fentes formées dans des raccords tubulaires à ferrule et, facultativement,
la mise en place comprend :
mettre en place chaque conducteur à l'intérieur de la fente de son raccord tubulaire à ferrule correspondant, avec sa structure de bille disposée contre une surface extérieure de son raccord tubulaire à ferrule correspondant.

4. Procédé selon la revendication 1, comprenant en outre :
pincer des parties de chaque raccord tubulaire à ferrule autour de la fente du raccord tubulaire à ferrule respectif pour courber les parties pour qu'elles se serrent autour de son conducteur correspondant et, facultativement,
réaliser les opérations de soudage comprend souder à la molette les parties courbées de chaque raccord tubulaire à ferrule autour de son conducteur correspondant.

5. Procédé selon la revendication 1, dans lequel des parties des fils de conducteur qui sont distales par rapport aux éléments de soudage sont entourées par un matériau isolant d'un corps de dérivation et, facultativement, le corps de dérivation est couplé à un élément de raccord agencé pour être raccordé électriquement à des bornes d'une dérivation de stimulation.

6. Procédé selon la revendication 1, comprenant en outre :
courber une ou plusieurs languettes de chaque élément de soudage pour fixer son fil conducteur correspondant en place avant de souder le fil conducteur à l'élément de soudage respectif, ou
réaliser une ou plusieurs opérations de soudage comprend : appliquer de l'énergie laser à l'élément de soudage, l'élément de soudage comprenant une ou plusieurs languettes agencées pour protéger le fil conducteur correspondant de l'élément de soudage vis-à-vis d'une exposition directe à l'écran laser appliqué,
réaliser la ou les opérations de soudage comprend : mettre en contact des électrodes d'un système de soudage par résistance contre des languettes d'un élément de soudage sans entrer directement en contact avec le fil conducteur correspondant de l'élément de soudage.

7. Procédé selon la revendication 1, comprenant en outre :
ajuster une orientation de languettes de l'un des éléments de soudage en fonction d'une orientation de son fil correspondant.

8. Procédé selon la revendication 1, dans lequel réaliser la ou les opérations de soudage comprend :
réaliser une première opération de soudage pour souder l'élément de soudage à sa broche de traversée correspondante ;
réaliser une seconde opération de soudage pour souder l'élément de soudage à son fil conducteur correspondant, ou
l'élément de soudage est soudé à sa broche de traversée correspondante et à son fil conducteur correspondant de manière sensiblement simultanée, ou
réaliser une ou plusieurs opérations de soudage amène chaque fil conducteur à fondre par conduction de chaleur provenant de son élément de soudage correspondant.

9. Procédé selon la revendication 1, dans lequel chaque élément de soudage comprend une surface incurvée agencée pour s'accoupler à une surface de chaque broche de traversée.

10. Procédé selon la revendication 9, dans lequel chaque élément de soudage comprend :
une indentation adjacente à une structure de languette pour recevoir un fil conducteur et, facultativement,
réaliser une ou plusieurs opérations de soudage comprend :
tenter une première opération de soudage sur une première languette de l'un des éléments de soudage pour souder à un fil conducteur correspondant au niveau d'une partie d'extrémité du fil conducteur ; et
réaliser une seconde opération de soudage, à l'achèvement infructueux de la première opération de soudage, sur une seconde languette de l'un des éléments de soudage pour souder au fil conducteur correspondant au niveau d'une partie relativement médiane du fil conducteur.

11. Générateur d'impulsions implantable comprenant :
un logement pour enfermer hermétiquement une circuiterie de génération d'impulsions et une circuiterie de commutation du générateur d'impulsions implantable ;
un ensemble traversée soudé au logement, l'ensemble traversée comprenant une pluralité de broches de traversée s'étendant depuis un espace intérieur dans le logement à un emplacement extérieur au logement, les broches de traversée étant couplées électriquement à la circuiterie de génération d'impulsions par l'intermédiaire de la circuiterie de commutation ;
un élément d'extension comprenant (i) un corps de dérivation s'étendant à l'opposé du logement, le corps de dérivation renfermant une pluralité de fils conducteurs enfermés à l'intérieur d'un matériau isolant, et
une pluralité d'éléments de soudage qui comprennent chacun une première partie soudée à une broche de traversée correspondante de l'ensemble traversée, **caractérisé par le fait que** chacun de la pluralité d'éléments de soudage comprend au moins une structure de languette entourant au moins partiellement et soudée à un fil conducteur correspondant du corps de dérivation ;
et **par le fait que** l'élément d'extension comprend (ii) une partie raccord électrique agencée pour recevoir des bornes d'une dérivation de stimulation, l'élément d'extension étant fixé de façon non amovible au logement.

12. Générateur d'impulsions implantable selon la revendication 11, dans lequel la première partie de chaque élément de soudage comprend une surface incurvée correspondant à un diamètre extérieur de la pluralité de broches de traversée.

13. Générateur d'impulsions implantable selon la revendication 11, dans lequel chaque élément de soudage comprend deux languettes disposées sur des côtés opposés du fil conducteur correspondant de l'élément de soudage respectif et, facultativement,
chaque élément de soudage comprend une indentation maintenant le fil conducteur correspondant de l'élément de soudage respectif et l'au moins une structure de languette est courbée sur le fil conducteur correspondant.

14. Générateur d'impulsions implantable selon la revendication 11, dans lequel chaque élément de soudage comprend deux languettes s'étendant dans une direction sensiblement radiale à l'opposé de la broche de traversée.

15. Générateur d'impulsions implantable selon la revendication 11, dans lequel au moins certains de la pluralité d'éléments de soudage sont orientés dans des directions différentes sur les broches de traversée de l'ensemble traversée.
